# EUROPEAN PATENT APPLICATION

(11) **EP 1 462 116 A1**
(43) Date of publication of application: **29.09.2004**
(21) Application number: 04251798.7
(22) Date of filing: 26.03.2004
(51) Int. Cl.: A61K 47/48

(54) **Composition for treating the gastrointestinal tract**

(30) Priority: 29.03.2003 GB 0307367; 21.05.2003 US 442372
(71) Applicant: Villitech SARL, 35800 Saint-Lunaire (FR)
(72) Inventor: Touchot, Nicholas, London SW4 8AE (GB)
(74) Representative: Peel, James Peter

(57) **Abstract**

The invention provides a physiologically acceptable polymeric material having one or more ligands capable of binding to a receptor in a human or animal gut, said receptor being selected from the group consisting of carbohydrate, aminoacid, lipid and peptide receptors, characterised in that the polymeric material is substantially incapable of being absorbed by the gut, which is useful in the treatment of obesity or in the improvement of the bodily appearance of a human or animal by reducing its weight.

## Description

### Field of Invention

The present invention is concerned with the treatment of the gastro-intestinal tract. More particularly, the present invention provides a composition and method capable of reducing the absorptive capabilities of the gastro-intestinal tract, facilitate weight loss and treat/prevent co-morbidities associated with overweight and obese individuals.

### Background of Invention

Overweight and obese individuals now represent ¼ to ½ of the adult population in western countries and put a very high burden on health care systems and resources, either directly or through associated conditions such as diabetes and heart diseases. Despite the development of multiple surgical and medical management approaches, there is still no easy, non-invasive treatment providing long-lasting treatment for obesity and its associated symptoms.

Overweight refers to an excess of body weight compared to set standards. The excess weight may come from muscle, bone, fat and/or body water. Obesity refers specifically to having an abnormally high proportion of body fat.

A number of methods are used to determine if an individual is overweight or obese. The simplest and easiest (even if not fully accurate) is the measurement of the Body Mass Index (BMI). BMI is found by dividing a person's weight in kilogram by height in meters squared. Many BMI calculators exist and tables have been published.

The US National Institutes of Health (NIH) identifies overweight as a BMI of 25-30 kg/m² and obesity as a BMI of 30kg/m² or over. These definitions are consistent with the recommendations of the World Health Organization and most other countries.

WHO Standard classification of obesity:

| | BMI | Risk of co-morbidities |
|---|---|---|
| Normal BMI | 18.5-24.9 | Average |

| *Overweight:* | | |
|---|---|---|
| Pre-Obese | 25.0-29.9 | Increased |
| Obesity class I | 30.0-34.9 | Moderate |
| Obesity class II | 35.0-39.9 | Severe |
| Obesity class III | > 40 | Very Severe |

Using these definitions, more than half of U.S. adults are overweight (BMI > 25) leading to a population of 97 million people. Nearly one quarter of U.S. adults are obese (BMI > 30) leading to a population of 40 millions.

Also very concerning is the prevalence of obesity in children. While there is no generally accepted definition for obesity as distinct from overweight, the prevalence of overweight is increasing for children and adolescent in the US with approximately 11% of the 6-17 year old population estimated to be overweight.

Prevalence of overweight and obesity is especially high in diabetics, hypertensive sufferers and individuals with high cholesterol levels

There are multiple ways for individuals to reduce weight and body fat, including diet, exercise, pharmaceutical and surgical approaches. Despite these various alternatives a growing number of individuals found it difficult to maintain a "healthy weight". This is due in part to lifestyle issues (only 22% of the US adults get the recommended regular physical activity), and in part to poor and inappropriate diet.

Some of the solutions which are used to treat the above conditions include surgery, drugs and diets.

Weight loss surgery for morbid obesity (bariatric surgery) is one of the fastest-growing areas of surgery today. Over the last two decades, approaches have evolved from fairly crude open surgery to a variety of laparoscopic procedures that usually involve creating a small gastric pouch ("restrictive procedure") limiting the amount of food a patient can eat or a bypass of the proximal end of the small intestine ("malabsortive procedure"), decreasing the ability for the food to be absorbed.

However, surgical approaches are also associated with a number of drawbacks including a requirement for general anesthesia, a stay in hospital, a long recovery time and complications arise in 5-10% of patients, potentially requiring an abdominal re-operation. Furthermore, obese patients usually have higher surgical risk than normal weight patients and could therefore be contra-indicated to surgery. As a result, surgical procedures are currently restricted to "morbid obesity" (WHO class III - BMI > 40) or to patients with BMI > 35 and with serious co-morbidities.

Several drugs have been developed for obesity. These drugs have gained medium acceptance especially for moderately obese patients with associated co-morbidities. However, many physicians feel that the medications are only mildly effective in treating obesity. Some physicians are hesitant to prescribe the mediations because of their side effects profiles

Approaches based on diet alone can be successful in the short term. However most patients who initially succeed with dieting regain the weight they have lost and sometimes more. Commonly the "cure rate" for morbid obesity is less than 5% with non-surgical methods.

Before the body can use the soluble products formed during digestive action, the nutrients must be absorbed through the lining of the digestive tract. Most of that absorption takes place in the small intestine. There are two routes of transport across the epithelium of the intestine: A transcellular route across the plasma membrane of epithelial cells and a paracellular route across tight junctions between epithelial cells.

The small intestine varies in length between 7 and 9 meters. It is divided in three main areas: The duodenum is a short (25-30 cm) section that receives secretions from the pancreas and the liver through the pancreatic and common bile ducts; the jejunum which represents about 40% of the small intestine and is responsible for the bulk of nutrient absorption; and the ileum which is the distal part of the small intestine and empties in the large intestine. The ileum plays a smaller part in absorption and is also important for water absorption and to serve as a buffer protecting the small intestine from the bacteria that flourish in the large intestine.

The structure of the small intestine is similar to other regions of the digestive tract, but the small intestine incorporates three features which account for its huge absorptive surface area: Mucosal folds: the inner surface of the small intestine is not flat, but thrown into circular folds; Villi: the mucosa forms multitudes of projections which protrude into the lumen and are covered with epithelial cells; and, Microvilli: the lumenal plasma membrane of absorptive epithelial cells is studded with densely-packed microvilli. The microvillus border of intestinal epithelial cells is referred to as the "brush border".

Large quantities of water are secreted in the lumen of the small intestine during the digestive process. Almost all of this water is also reabsorbed in the small intestine in response to osmotic gradients. The small intestine is also a very dynamic organ. The wall of the intestine contains two layers of muscle structures (Muscularis externa and Muscularis interna), which control intestinal motility.

It is within the small intestine that the final stages of enzymatic digestion occur, liberating small molecules capable of being absorbed. The small intestine is also the sole site in the digestive tract for absorption of amino acids and monosaccharides. Most lipids are also absorbed in this organ. All of this absorption and much of the enzymatic digestion takes place on the surface of small intestinal epithelial cells, and to accommodate these processes, a huge mucosal surface area is required. After crossing the epithelium, most of these molecules diffuse into a capillary network inside the villus, and hence into systemic blood. Some molecules, fats in particular, are transported not into capillaries, but rather into the lymphatic vessel, which drains from the intestine and rapidly flows into blood via the thoracic duct.

Some molecules, water for instance, are transported by both transcellular and paracellular routes. In contrast, the tight junctions are impermeable to large organic molecules from the diet (e.g. amino acids and glucose). These types of molecules are transported exclusively by the transcellular route, and only because the plasma membrane of the absorptive enterocytes is equipped with transporter molecules that facilitate entry into and out of the cells.

As ingesta travels through the intestine, it is sequentially exposed to regions having epithelia with very different characteristics. This diversity in function results from differences in phenotype of the enterocytes - that is, the number and type of transporter molecules they express in their plasma membrane and the structure of the tight junctions they form.

Absorption of each of the major food groups shall now be discussed separately:
(i) Carbohydrates, after digestion to monosaccharides,
(ii) Proteins, after digestion to small peptides and amino acids, and
(iii) Neutral fat, after digestion to monoglyceride and free fatty acids.

Simple sugars are the predominant carbohydrate absorbed in the digestive tract. Monosaccharides, however, are only rarely found in normal diets. Rather, they are derived by enzymatic digestion of more complex carbohydrates within the digestive tract.

Particularly important dietary carbohydrates include starch and disaccharides such as lactose and sucrose. None of these molecules can be absorbed for the simple reason that they cannot cross cell membranes unaided and, unlike the situation for monosaccharides, there are no transporters to carry them across.

Polysaccharides and disaccharides must be digested to monosaccharides prior to absorption and the key players in these processes are the brush border hydrolases, which include maltase, lactase and sucrase. Dietary lactose and sucrose are "ready" for digestion by their respective brush border enzymes. Starch is first digested to maltose by amylase in pancreatic secretions and, in some species, saliva.

Dietary lactose and sucrose, and maltose derived from digestion of starch, diffuse in the small intestinal lumen and come in contact with the surface of absorptive epithelial cells covering the villi where they engage with brush border hydrolases:
(i) maltase cleaves maltose into two molecules of glucose
(ii) lactase cleaves lactose into a glucose and a galactose
(iii) sucrase cleaves sucrose into a glucose and a fructose

Glucose, galactose and fructose are each taken into the enterocyte by facilitated diffusion. Glucose and galactose utilize the same transporter, while the fructose transporter is a separate entity. Absorption of glucose, or any molecule for that matter, entails transport from the intestinal lumen, across the epithelium and into blood. The transporter that carries glucose and galactose into the enterocyte is the sodium-dependent hexose transporter, known more formally as SGLUT-1. As the name indicates, this molecule transports both glucose and sodium into the cell.

The essence of transport by the sodium-dependent hexose transporter involves a series of conformational changes induced by binding and release of sodium and glucose, and can be summarized as follows:
(i) the transporter is initially oriented facing into the lumen - at this point it is capable of binding sodium, but not glucose
(ii) sodium binds, inducing a conformational change that opens the glucose-binding pocket
(iii) glucose binds and the transporter reorients in the membrane such that the pockets holding sodium and glucose are moved inside the cell
(iv) sodium dissociates into the cytoplasm, causing glucose binding to destabilize
(v) glucose dissociates into the cytoplasm and the unloaded transporter reorients back to its original, outward-facing position.

Dietary proteins are, with very few exceptions, not absorbed. Rather, they must be digested into amino acids or di- and tripeptides first. There are two sources which secrete proteolytic enzymes into the lumen of the digestive tract:
(i) the stomach secretes pepsinogen, which is converted to the active protease pepsin by the action of acid.
(ii) the pancreas secretes a group of potent proteases, chief among them trypsin, chymotrypsin and carboxypeptidases.

Through the action of these gastric and pancreatic proteases, dietary proteins are hydrolyzed within the lumen of the small intestine predominantly into medium and small peptides (oligopeptides).

The brush border of the small intestine is equipped with a family of peptidases. Like lactase and maltase, these peptidases are integral membrane proteins rather than soluble enzymes. They function to further the hydrolysis of lumenal peptides, converting them to free amino acids and very small peptides. These end products of digestion, formed on the surface of the enterocyte, are ready for absorption.

The mechanism by which amino acids are absorbed is conceptually identical to that of monosaccharides. The lumenal plasma membrane of the absorptive cell bears at least four sodium-dependent amino acid transporters - one each for acidic, basic, neutral and amino acids. These transporters bind amino acids only after binding sodium. The fully loaded transporter then undergoes a conformational change that dumps sodium and the amino acid into the cytoplasm, followed by its reorientation back to the original form.

There is virtually no absorption of peptides longer than three amino acids. However, there is abundant absorption of di- and tripeptides in the small intestine. Once inside the enterocyte, the vast bulk of di- and tripeptides are digested into amino acids by cytoplasmic peptidases and exported from the cell into blood.

The bulk of dietary lipid is neutral fat or triglyceride, composed of a glycerol backbone with each carbon linked to a fatty acid. Additionally, most foodstuffs contain phospholipids, sterols like cholesterol and many minor lipids, including fat-soluble vitamins. In order for the triglyceride to be absorbed, two processes must occur:
(i) Large aggregates of dietary triglyceride, which are virtually insoluble in an aqueous environment, must be broken down physically and held in suspension - a process called emulsification.
(i) Triglyceride molecules must be enzymatically digested to yield monoglyceride and fatty acids, both of which can efficiently diffuse into the enterocyte.

These transformations are facilitated by bile salts and pancreatic lipase, both of which are mixed with chyme and act in the lumen of the small intestine. Bile salts play their first critical role in lipid assimilation by promoting emulsification. As derivatives of cholesterol, bile salts have both hydrophilic and hydrophobic domains. On exposure to a large aggregate of triglyceride, the hydrophobic portions of bile salts intercalate into the lipid, with the hydrophilic domains remaining at the surface. Such coating with bile salts aids in breakdown of large aggregates or droplets into smaller and smaller droplets.

Hydrolysis of triglyceride into monoglyceride and free fatty acids is accomplished predominantly by pancreatic lipase. The activity of this enzyme is to clip the fatty acids at positions 1 and 3 of the triglyceride, leaving two free fatty acids and a 2-monoglyceride.

As monoglycerides and fatty acids are liberated through the action of lipase, they retain their association with bile salts and complex with other lipids to form micelles. As the ingesta is mixed, micelles contact the brush border and the lipids, including monoglyceride and fatty acids, are absorbed.

Lipids are absorbed by a mechanism distinctly different from that of monosaccharides and amino acids. A considerable fraction of the fatty acids also enter the enterocyte via a specific fatty acid transporter protein in the membrane.

### Summary of Invention

In one aspect of the present invention, there is provided a physiologically acceptable polymeric material which is substituted by one or more ligands capable of binding to a receptor in a human or animal gut, said receptor being selected from the group consisting of carbohydrate, amino-acid, lipid and peptide receptors, characterised in that the polymeric material is substantially incapable of being absorbed by the gut.

In a second aspect of the present invention, there is provided a composition comprising a polymeric material according to the invention and a carrier.

In a further aspect of the present invention, there is provided a method of treatment of obesity in a human or animal which method comprises administering to a human or animal in need of such treatment a therapeutically effective amount of a polymeric material according to the invention and/or of a composition according to the invention. Preferably, the composition is targeted at the small intestine, preferably the jejunum and/or ileum.

According to a further aspect of the invention, there is provided a method of reducing the weight of a human or animal to improve its bodily appearance which method comprises administering to a human or animal in need of such treatment a cosmetically effective amount of a polymeric material according to the invention and/or a composition according to the invention.

The polymeric material is preferably for use in a method of medical treatment, more preferably for use in the treatment of obesity. According to a further aspect of the invention, there is provided use of the polymeric material according to the invention and/or of the composition according to the invention in a method of cosmetic treatment to improve the bodily appearance of a human or animal by reducing its weight. According to another aspect of the invention, there is provided use of the polymeric material according to the invention and/or of the composition according to the invention in the manufacture of a medicament for use in the treatment of obesity.

The composition of the present invention is preferably a pharmaceutical composition, preferably for use in a method of cosmetic or medical treatment, more preferably for use in the treatment of obesity and/or improving the bodily appearance by reduction of weight.

The term "incapable of being absorbed by the gut" is intended to mean that the polymeric material is not absorbed by the small intestine via the transcellular or paracellular routes under normal physiological conditions.

The present invention aims to significantly reduce the absorptive area of the small intestine by the creation of a physical barrier between the absorptive area and the chyme. This may be effected in a number of ways, including linking the villi and microvilli to each other, thereby restricting the surface area that may contact the chyme and/or by coating the surface area of the villi and microvilli, thus restricting the available contact surface. Effectively, the present invention provides a polymeric material which adsorbs to the intestine wall but is not absorbed thereby.

The villi form an extremely closely packed brush border at the surface of the intestine. It would require a large amount of material to coat the entire surface of the villi, and substantially more to coat the microvilli. The brush border structure provides the opportunity to modify drastically the absorptive area of the small intestine by linking the individual villi and microvilli to one another. This results in mechanically closing a significant surface area that is then prevented from contacting the chyme. Consequently, the corresponding surface area is no longer able to absorb nutrients and the overall absorptive capabilities of the intestine (both transcellular and paracellular) are significantly reduced. This, in turn, leads to an energy inbalance, leading to weight loss and allowing prevention and treatment of obesity and an obesity associated co-morbidity.

To achieve the adhesion of villi/microvilli to each other, the present invention provides a polymeric material or a composition which enables the crosslinking of the villi or microvilli structures to one another.

The present invention shall now be described with reference to the figures which show:
Figure 1 shows how the composition according the present invention links two features of the small intestine.
Figure 2 shows an alternative view of the composition of the present invention linking two features of the intestine.

### Detailed Description of the Invention

The polymeric material according to the invention is preferably a substantially biologically inert polymeric material having one or more ligands, which may be the same or different, appended thereto, each of said ligands being specific for binding to a nutrient substrate receptor of the gut epithelium. It is preferable that the polymeric material chosen for the practice of the invention described herein is non-absorbable or substantially non-absorbable in the patient's body.

The polymeric material is preferably substantially insoluble in aqueous media at the natural pH of a small intestine of a human or animal. Preferably, the polymeric material-linker-ligand or the polymeric material-ligand components of the composition, as defined below, are incapable of being absorbed by the gut.

The polymeric material may be a material that is substantially stable in a human or animal gastro-intestinal tract or may be one which degrades over a predetermined period of time in the gastro-intestinal tract. Thus, the polymeric material may remain bound to the villi or microvilli until the gut sheds the cells to which the polymeric material is bound. This provides an elegant reversible system that allows the villi or microvilli to be bound for a period of time determined by the life of the epithelial cells lining the villi/microvilli in the gut.

Alternatively, the polymeric material may comprise one or more structures or bonds which are unstable in the gastro-intestinal tract. For example, the polymeric material may be pH sensitive, and may thus be designed to degrade slowly in the alkaline pH of the small intestine.

The degradation product of the polymeric material or its ligand components may be absorbable by the intestine. Thus the polymeric material and associated ligands remain substantially non-absorbable until and/or unless they degrade, when they may be absorbable. Preferably, the composition of the present invention has a residence time in the intestine in the range of 1 hour to 2 months, more preferably 5 hours to 1 month, more preferably 10 hours to 2 weeks, more preferably greater than 24 hours, before substantial degradation of the polymer and/or ligands and/or linker take place. Substantial degradation means greater than 5%, more preferably greater than 10%, more preferably greater than 25% by weight lost through degradation of a discrete polymer-ligand molecule.

Residence time refers only to the polymeric material which adsorbs to the intestines, not to that which passes through the GI tract without adsorbing.

The composition and/or the polymeric material of the present invention is preferably particulate. The mean particle diameter is preferably in the range of 0.01µm-100µm, more preferably 0.05µm-50µm, more preferably 0.1µm-25µm, most preferably less than 1µm.

Preferably the polymeric material is synthetic, although a natural polymer or a derivative thereof may be utilised, for example a cellulosal or liposomal material. The polymeric material may be in the form of a substantially unbranched or branched polymeric backbone. Alternatively, dendritic polymeric material may be provided. The polymeric material may be in the form of a bead, microbead, particle or globular structure.

An exemplary polymeric material is preferably selected from the group consisting of an epoxy resin, fluoropolymer, phenolic resin, melamine resin, polyacetal, polyacetylene, polyacrylic, polyalkylene, polyalkenylene, polyamic acid, polyamide, polyamine, polyanhydride, polyarylene, polybenzyl, polycarbodiimide, polycarbonate, polycarbosilane, polycarborane, polydiene, polyester, polyurethane, polyetherketone, polyether, polyimidazole, polyimine, polyimide, polyisocyanate, polyisocyanurate, polyketone, polyolefin, polyoxide, polyoxyalkylene, polyoxyarylene, polyphenyl, polyquinoline, polysilane, polysiloxane, polyurea, polyvinylacetal, polyacetal, polysaccharide and a copolymer and a derivative thereof.

A particularly preferred polymeric material includes a polyamine, polybutadiene, polyalkyleneglycol, polystyrene, polyalkyl(alk) acrylate, polysaccharide, polyhydroxyalkylmethacrylate, hydroxyalkylmethylcellulose, and a polymer of acrylic and methacrylic acid, maleic polymer, including a copolymer and a derivative thereof.

In a particularly preferred embodiment, the polymeric material comprises a polymer selected from functionalised polyethyleneglycol, functionalised polypropyleneglycol, melamine resins, melamine-formaldehyde resins, cellulose, functionalised polystyrene, poly(hydroxyalkyl(alk)acrylamide) and a mixture or copolymer thereof.

A particularly preferred functionalised PEG polymer includes PEG-acrylate, PEG-aldehyde, PEG-cyanoacetate, PEG-dipalmitoyl phosphatidylethanolamine, PEG-distearoyl phosphatidylethanolamine, PEG-epoxide, PEG-hydrazide, PEG-isocyanate, PEG-maleimide, PEG-methacrylate, PEG-nitrophenyl carbonate, PEG-orthopyridyl disulfide, PEG-silane, PEG-sulfhydryl, PEG-succinimidyl glutarate, PEG-succimidyl succinate, PEG-succinic acid, PEG-tresylate. These may be readily obtained from a number of commercial sources including SunBio and BioVectra.

A particularly preferred functionalised styrene polymer includes polystyrene-acrylate, polystyrene-aldehyde, polystyrene-cyanoacetate, polystyrene-dipalmitoyl phosphatidylethanolamine, polystyrene-distearoyl phosphatidylethanolamine, polystyrene-epoxide, polystyrene-hydrazide, polystyrene-isocyanate, polystyrene-maleimide, polystyrene-methacrylate, polystyrene-nitrophenyl carbonate, polystyrene-orthopyridyl disulfide, polystyrene-silane, polystyrene-sulfhydryl, polystyrene-succinimidyl glutarate, polystyrene-succimidyl succinate, polystyrene-succinic acid, polystyrene-tresylate. Such a polymer may be obtained from a number of commercial sources including Glycopep.

Melamine is preferred in the form of a resin particle having a diameter of from 1-25µm. A functionalised polystyrene particle having a diameter of from 1-1000µm is also preferred. Such compounds may be obtained from Microparticles GmbH.

The term "copolymer" is used herein to mean mixed polymers which contain more than one homopolymer. "Derivitisable group" means a reactive group which may be reacted to functionalise the polymer. "Derivative" means a polymer or compound that has been chemically modified, preferably via modification of a derivatisable group. A preferred derivitisable group includes an olefinic, carboxyl, amine, carbonyl, imino, hydroxyl group or a protected analogue thereof. Protection is effected by reaction with an appropriate protecting group.

The term "protecting group" refers to a group in a multifunctional compound that may temporarily activate or temporarily block a reactive site wherein a chemical reaction is to be carried out selectively at a reactive site. The reactive site may be other than the site occupied by the "protecting group". The protecting group referred to, in the context of the present invention, is a commonly known protecting group including, but not limited to n-succinimidyl, pentachlorophenyl, pentafluorophenyl, para-nitrophenyl, dinitrophenyl, n- phthalimido, n-norbornyl, cyanomethyl, isopropylidenyl, pyridyl, trichlorotriazine, 5-chloroquinilino, n- (9-fluorenylmethoxycarbonyl) (fmoc), carbobenzyloxy (cbz), 1-(4,4-dimethyl-2,6-dioxocyclohexylidene) ethyl (dde) and imidazolyl.

A preferred polymeric materials has a molecular weight in the range of 1,000-500,000, more preferably 5,000-250,000, more preferably greater than 50,000. A charged or crosslinked polymer or a polymer which is insoluble under the physiological conditions of the small intestine (such as by crosslinking), eliminate or significantly reduce transportation of the polymer across the gut wall, and is preferred.

Another type of preferred polymeric material includes a polymer that is adapted to swell at the pH value of the small intestine. This provides an opportunity to deliver the polymer to the desired site in the intestine, for example, an inter-villi space, and allow the polymer to swell in order to block the inter-villi space, and/or bring the ligand into close proximity with a receptor.

The ligand is preferably a nutrient receptor substrate or a precursor or derivative thereof. For example, the ligand is preferably a member of a binding pair. The ligand should be recognised and selectively bound by a receptor in the small intestine. Thus, any chemical derivatisation of the ligand, for example to attach it to the polymeric material, should retain the "identity" of the ligand such that it is still recognised by the nutrient receptor.

The ligand is preferably incorporated into the polymeric material by covalently bonding the receptor substrate to the polymeric material, such as to the polymer backbone and/or to pendant functional groups on the polymer backbone, by way of a covalent bond. The ligand can be covalently bonded to the polymeric material by way of a chemical linker.

The ligand is preferably spacially remote from the polymeric material. This minimises steric or chemical interference between the ligand and its receptor. Further, the ligand is preferably relatively sterically or chemically unhindered. This allows the nutrient receptor to recognise the molecule and reversibly or irreversibly bind the ligand. Preferably, the bond between the ligand and the polymeric material is maintained during and subsequent to said receptor binding. Alternatively, the bond between the ligand and the polymeric material may be degradable, preferably selectively degradable, for example by changes in pH.

The ligand is preferably a covalently bound receptor substrate. In particular, the ligand is preferably selected from glucose, galactose, fructose, a monoglyceride, fatty acids, synthetic amino acids, lysine, arginine, histidine, aspartic acid, glutamic acid, asparagine, glutamine, serine, threonine, tyrosine, glycine, alanine, valine, leucine, isoleucine, proline, phenylalanine, methionine, tryptophan, and cystein and a derivative thereof. An ester, amide or glycosidic linkage is a particularly preferred linkage for bonding the ligand to the polymeric material.

It should be understood that the above definitions are intended to cover structural and optical isomeric forms of the ligand utilised in the present invention. For example, pyranose and furanose forms of monosaccharide are envisaged.

Preferably, the polymeric material comprises a plurality of ligands, preferably 2-1000000, more preferably 10-500000, more preferably 50-100000, most preferably greater than 100 ligands.

In order to chemically connect the ligand to the polymeric material, selected functional groups in both chemical entities may be used to covalently bind them through a specific chemical linker. Preferred functional groups in nutrient receptor substrates, for example, carbohydrates, include hydroxy, amino, mercapto, carbonyl, carboxyl, and amido groups. An ester, amide or glycosidic linkage is a particularly preferred way of bonding the linker to the ligand and/or to the polymeric material.

The linker may be hydrolytically labile or stable, preferably hydrolytically stable. A hydrolytically stable linkage is a linkage which is stable in water and does not react with water at useful pHs for an extended period of time, potentially indefinitely. A hydrolytically unstable linkage is a linkage that reacts with water, typically causing degradation of a polymer and/or release of a polymer substituent. The time it takes to degrade a crosslinked polymeric structure is referred to as the rate of hydrolysis and is usually measured in terms of its half life. The linker is preferably a multi-functional compound which is capable of covalently linking the ligand to the polymeric material.

Preferred reagents for linking the polymeric material to the linker and/or ligand, and for linking the linker to the ligand include:
Reacting an amine functional linker or ligand with a polymer comprising a group selected from activated esters, carboxylic acids, isocyanates and aldehydes;
Reacting a carboxyl functional linker or ligand with a polymer comprising a group selected from hydroxyl and amine; and,
Reacting a hydroxyl functional linker or ligand with a polymer comprising a group selected from p-nitrophenyl carbonate and isocyanate.

A preferred linker is a natural or synthetic amino acid and/or peptide, diamine, dicarboxylic acid, diol, hydroxyalkylamine, thiol, aldehyde and ketone. A particularly preferred linker comprises a difunctional alkyl, aryl or alkenyl group. Obviously there are many more possible functional groups and chemical bonds available for this particular purpose.

It should be understood that a number of the coupling reactions listed above may require the provision of a coupling agent to catalyse or otherwise facilitate the reaction.

The composition of the present invention optionally further includes a pharmaceutically acceptable additive and/or excipient. A suitable additive and/or excipient includes, without limitation, a detackifier, anti-foaming agent, buffering agent, polymer, antioxidant, preservative, chelating agent, viscomodulator, tonicifier, flavorant, colorant, odorant, opacifier, suspending agent, binder, filler, plasticizer, lubricant, or a mixture thereof.

The composition of the present invention is preferably administered orally. The composition is preferably in a solid or liquid form.

The composition of the present invention can be processed and prepared according to a conventional technique known to those skilled in the art, such as lyophilization, encapsulation, compression, melting, extrusion, balling, drying, chilling, molding, spraying, spray congealing, coating, comminution, mixing, homogenization, sonication, cryopelletization, spheronization, and granulation, to produce the desired dosage form. Processing techniques such as size reduction, co-precipitation, coacervation, lyophilizing, spray drying, eutectic mixing and solid solutioning are particularly useful for making the composition.

The composition of the invention may be in the form of a tablet, lozenge, pill, troche, capsule, soft-gel capsule, sachet or other combining vehicle, elixir, powder, including lyophilised powder, solution, granule, suspension, emulsion, syrup or tincture. A slow-release, or delayed-release, form may also be prepared, for example in the form of a coated particle, multi-layer tablet or microgranule. The composition may also be presented in a compliance-enhancing blister pack.

The composition can be administered in one dose, continuously or intermittently throughout the course of treatment. Methods of determining the most effective means and dosage of administration are well known to those of skill in the art.

The targeting of ligands to desired locations in the alimentary canal can be complicated. Various factors must be taken into consideration for delivery to desirable areas of the alimentary canal. Each segment of the alimentary canal has distinct features which may hinder or favour permeation of receptor substrates across the membrane.

Delivery of a composition according to the present invention to a site beyond the stomach is especially desirable for a polymeric material that is destroyed by the acid conditions or enzyme of the stomach, or for a polymeric material that causes local irritation in the stomach. However, targeting to other areas of the small intestine may involve several additional systems. The low stomach pH and presence of gastric enzymes have led to forms in which the composition is provided with an enteric coating. Coating is accomplished with a selectively soluble substance, and protects a composition and its components from inactivation by gastric enzymes and/or low pH.

The enteric coating is typically although not necessarily a polymeric material. Preferred enteric coating materials comprise bioerodible, gradually hydrolyzable and/or gradually water-soluble polymers. The "coating weight" or relative amount of coating material per dosage form, generally dictates the time interval between ingestion and drug release. Any coating material should be applied to a sufficient thickness such that the entire coating does not dissolve in the gastrointestinal fluids at pH below about 5, but does dissolve at pH about 5 and above.

Suitable enteric coating materials include, but are not limited to: cellulosic polymers such as hydroxypropyl cellulose, hydroxyethyl cellulose, hydroxypropyl methyl cellulose, methyl cellulose, ethyl cellulose, cellulose acetate, cellulose acetate phthalate, cellulose acetate trimellitate, hydroxypropylmethyl cellulose phthalate, hydroxypropylmethyl cellulose succinate and carboxymethylcellulose sodium; acrylic acid polymers and copolymers, preferably formed from acrylic acid, methacrylic acid, methyl acrylate, ammonio methylacrylate, ethyl acrylate, methyl methacrylate and/or ethyl methacrylate (e.g., those copolymers sold under the tradename "Eudragit"); vinyl polymers and copolymers such as polyvinyl pyrrolidone, polyvinyl acetate, polyvinylacetate phthalate, vinylacetate crotonic acid copolymer, and ethylenevinyl acetate copolymers; and shellac (purified lac).

The composition according to the invention optimally includes a bioadhesive to prolong intestinal transit, as in a buccal delivery system. The adhesion to the intestinal mucosa takes place either by mechanical interlocking or other mechanisms (Longer, M. A., et. al., Pharm. Int. 7:114-7 (1986)).

The composition of the present invention may be used to therapeutically treat an individual by delivering a biologically active compound to the gut, for example a drug. It should be stressed that the treatment of ailments and diseases, whether obesity associated co-morbidities which may be treated simply by reducing nutrient absorption in the gut, or by the delivery of a drug to the gut, may both be achieved using a composition and method according to the present invention.

In this embodiment, drug delivery is preferably achieved by incorporation of a drug into the polymeric material. Alternatively, a drug may be attached to the polymeric material, for example directly or via a linker molecule as defined above. Alternatively, a drug may simply be coated on the surface of the polymeric material utilised in the present invention.

For the drug delivery aspect of the present invention, the polymeric material used to incorporate the drug may be porous, degradable and/or swellable so as to facilitate drug delivery.

As used herein, the term "drug" refers to chemical or biological molecules providing a therapeutic, diagnostic, or prophylactic effect in vivo.

The present invention has particular utility in the area of drug delivery as microenvironments are created when villi or microvilli are crosslinked. Effectively, a closed local environment may be produced via the crosslinking. Consequently, localised concentration of drug may be provided in the regions of crosslinked villi and microvilli. This has obvious advantages in that lower quantities of drug need be applied to the area being treated. Other methods of local delivery to the intestine suffer the problems of the drug being washed away via the natural motility of the intestine.

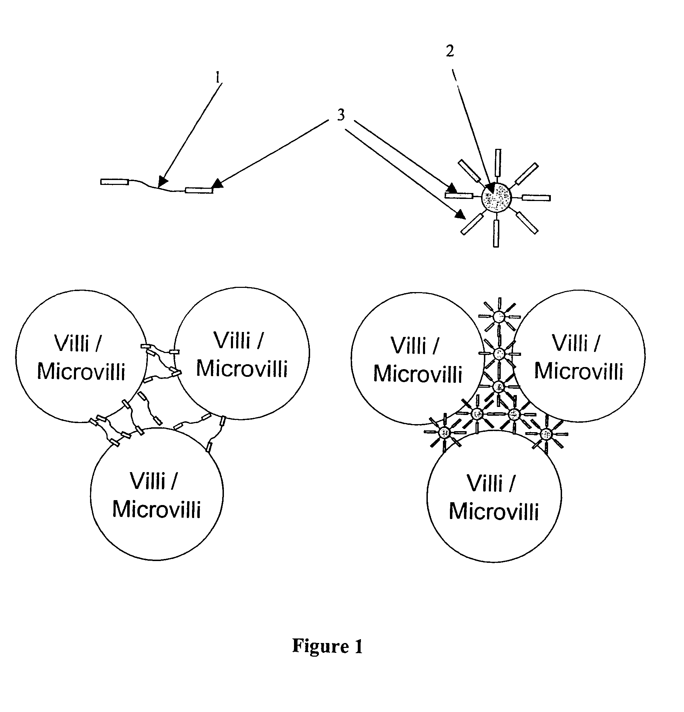

With regards to Figure 1, a filamentous polymer 1 is shown in the upper left. The polymer 1 comprises ligands 3 attached thereto. On the upper right of figure 1, a microbead 2 is shown, having a number of ligands 3 attached to the surface thereof. The lower half of Figure 1 shows how the filamentous polymer and the microbead respectively crosslink villi and/or microvilli located in the intestine.

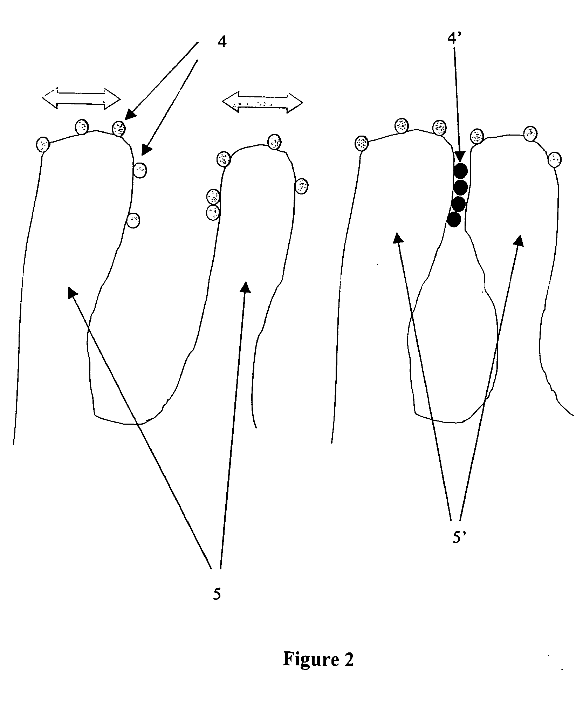

Figure 2 illustrates the side view of villi/microvilli 5, 5'. On the left of Figure 2, the villi/microvilli are not crosslinked, as indicated by the block arrows. Particulate material 4 from the composition according to the present invention is shown adhered to the surface of the villi/microvilli. The natural motility of the villi/microvilli causes the structures 5 to move. On the right of Figure 2, villi/microvilli 5' are shown crosslinked to one another via particulate material 4' according to the composition of the present invention.

### Examples

In a particularly preferred embodiment of the present invention, monosaccharide ligands are attached to a functionalised polymeric material such as amine functional polyethylene glycol (PEG---NH₂) or melamine. This is preferably achieved in an analogous method to that disclosed in US Patent No. 5,723,589, as summarised in schemes 1 and 2 below.

Essentially, scheme 1 shows a short difunctional linker molecule, in this case hexane-1,2-diol being activated, protected and functionalised with a molecule of isopropylidene glucofuranose and subsequently deprotected to form a glucofuranose-alkanol compound. This may be reacted with a functionalised polymeric material, for example a carboxylate functionalised polymeric compound, to form an ester linked, glucose functionalsed polymer. Alternatively, the hydroxy group of the alkanol moiety may be converted to a carboxylic acid group, via an aldehyde group. The resulting compound may be reacted with an amine functionalised or hydroxy functionalised polymeric material to form a glucose substituted polymeric material. The furanose form is shown being converted into a pyranose form and being deprotected. The specific conditions and reagents for these reactions are discussed in the above mentioned US Patent No. 5,723,589.

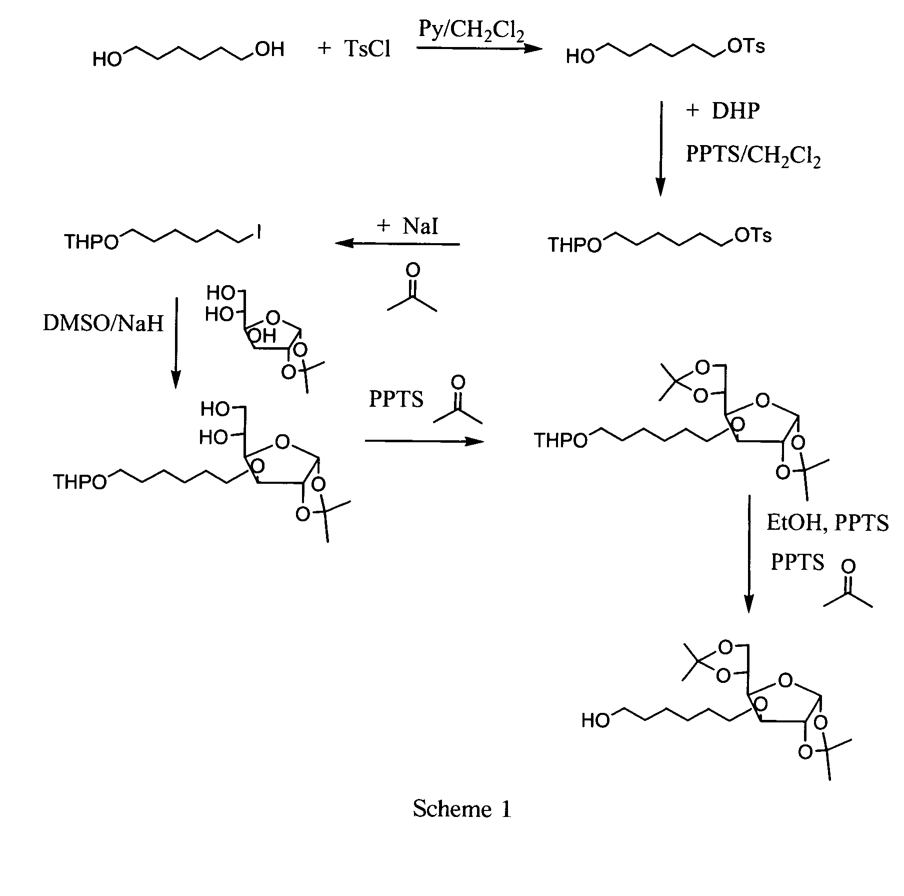

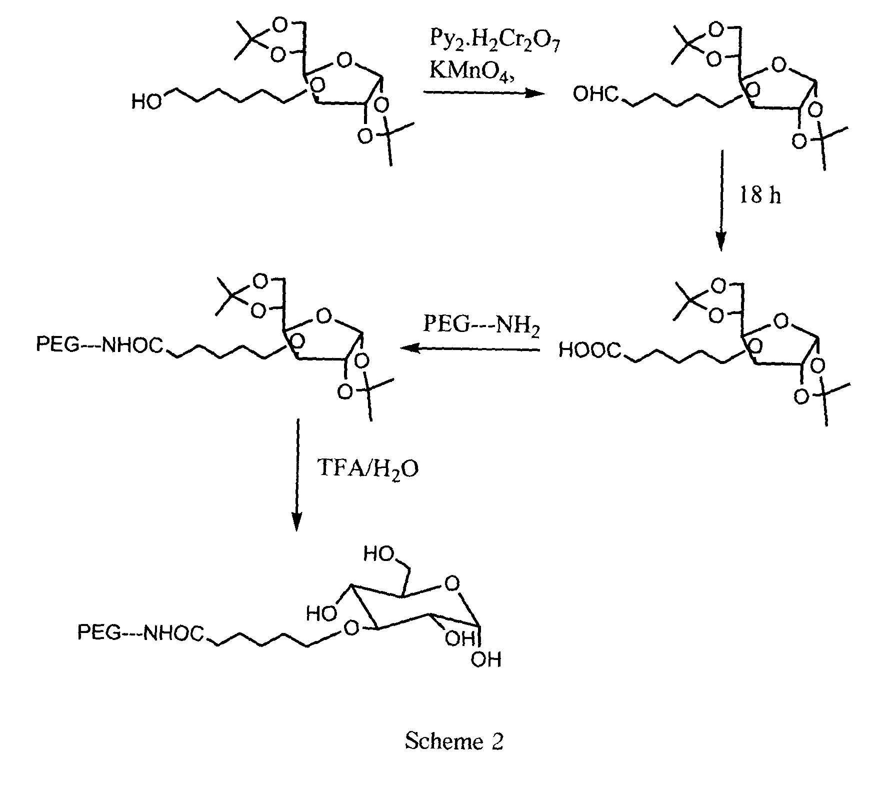

## Claims

1. A physiologically acceptable polymeric material having one or more ligands capable of binding to a receptor in a human or animal gut, said receptor being selected from the group consisting of carbohydrate, amino-acid, lipid and peptide receptors, **characterised in that** the polymeric material is substantially incapable of being absorbed by the gut.

2. A polymeric material as defined in Claim 1 which is substantially insoluble in aqueous media at the physiological pH of the small intestine.

3. A polymeric material as defined in Claim 1 or Claim 2 which is particulate.

4. A polymeric material as defined in Claim 3, wherein the mean particle diameter is in the range of 0.01µm-100µm, more preferably 0.05µm-50µm, more preferably 0.1µm-25µm, most preferably less than 1µm.

5. A polymeric material as defined in any one of the preceding Claims which is selected from the group consisting of epoxy resin, fluoropolymer, phenolic resin, melamine resin, polyacetal, polyacetylene, polyacrylic, polyalkylene, polyalkenylene, polyamic acid, polyamide, polyamine, polyanhydride, polyarylene, polybenzyl, polycarbodiimide, polycarbonate, polycarbosilane, polycarborane, polydiene, polyester, polyurethane, polyetherketone, polyether, polyimidazole, polyimine, polyimide, polyisocyanate, polyisocyanurate, polyketone, polyolefin, polyoxide, polyoxyalkylene, polyoxyarylene, polyphenyl, polyquinoline, polysilane, polysiloxane, polyurea, polyvinylacetal, polyacetal, polysaccharide and a copolymer and a derivative thereof.

6. A polymeric material as defined in any one of the preceding Claims which is selected from the group consisting of a polyamine, polybutadiene, polyalkyleneglycol, polystyrene, polyalkyl(alk) acrylate, polysaccharide, polyhydroxyalkylmethacrylate, hydroxyalkylmethylcellulose, and a polymer of acrylic and methacrylic acid, maleic polymer, a copolymer and a derivative thereof.

7. A polymeric material as defined in any one of the preceding Claims which is selected from the group consisting of functionalised polyethyleneglycol, functionalised polypropyleneglycol, melamine resins, melamine-formaldehyde resins, cellulose, functionalised polystyrene, poly(hydroxyalkyl(alk)acrylamide) and a mixture and a copolymer thereof.

8. A polymeric material as defined in any one of the preceding Claims, wherein the molecular weight of the polymeric material is in the range of 1,000-500,000, preferably 5,000-250,000, most preferably greater than 50,000.

9. A polymeric material as defined in any one of the preceding Claims which is swellable in aqueous solution.

10. A polymeric material as defined in any one of the preceding Claims, wherein the ligand is a nutrient receptor substrate or precursor or derivative thereof.

11. A polymeric material as defined in Claim 10, wherein the ligand is selected from the group consisting of glucose, galactose, fructose, a monoglyceride, a fatty acid, synthetic amino acid, lysine, arginine, histidine, aspartic acid, glutamic acid, asparagine, glutamine, serine, threonine, tyrosine, glycine, alanine, valine, leucine, isoleucine, proline, phenylalanine, methionine, tryptophan, and cystein and a derivative thereof.

12. A polymeric material as defined in any one of the preceding Claims, wherein the polymeric material comprises a plurality of ligands, preferably 2-1,000,000, more preferably 10-500,000, more preferably 50-100,000, most preferably, greater than 100 ligands.

13. A polymeric material as defined in any one of the preceding Claims, wherein the ligand is covalently attached to the polymeric material by a linker molecule.

14. A polymeric material as defined in Claim 13, wherein the linker is selected from the group consisting of a natural or synthetic amino acid and a peptide, diamine, dicarboxylic acid, diol, hydroxyalkylamine, thiol, aldehyde and a ketone.

15. A polymeric material as defined in any one of the preceding Claims, for use in a method of therapy, preferably for use in the treatment of obesity.

16. A composition comprising a polymeric material as defined in any one of the preceding Claims in association with a carrier.

17. A composition as defined in Claim 16 which is a pharmaceutical composition, preferably for use in a method of medical treatment, more preferably for use in the treatment of obesity

18. A composition as defined in Claim 16 which is for use in a method of cosmetic treatment, preferably for use in improving the bodily appearance of a human or animal by reduction of weight.

19. A method of treatment of obesity in a human or animal which method comprises administering to a human or animal in need of such treatment a therapeutically effective amount of a polymeric material as defined in any one of Claims 1 to 15 and/or of a composition as defined in any one of Claims 16 to 18.

20. A method according to claim 19, wherein villi and/or microvilli in the intestine of the human or animal are crosslinked to one another, thereby reducing the effective absorptive area of the intestine.

21. A method as defined in Claim 19 wherein the composition is targeted at the small intestine, preferably the jejunum and/or ileum.

22. A method of reducing the weight of a human or animal to improve its bodily appearance which method comprises administering to a human or animal in need of such treatment a cosmetically effective amount of a polymeric material as defined in any one of Claims 1 to 15 and/or of a composition as defined in any one of Claims 16 to 18.

23. Use of the polymeric material as defined in any one of Claims 1 to 15 and/or of a composition as defined in any one of Claims 16 to 18 in a method of cosmetic treatment to improve the bodily appearance of a human or animal by reducing its weight.

24. Use of the polymeric material as defined in any one of Claims 1 to 15 and/or of a composition as defined in any one of Claims 16 to 18 in the manufacture of a medicament for use in the treatment of obesity.
